# FASCICULE DE BREVET EUROPEEN

(11) **EP 0 950 048 B1**
(45) Date de publication et mention de la délivrance du brevet: **27.06.2001**
(21) Numéro de dépôt: 97951323.1
(22) Date de dépôt: 12.12.1997
(51) Int. Cl.: C07D 211/70, C07D 401/04, A61K 31/445

(54) **1-PHENYLALKYL-1,2,3,6-TETRAHYDROPYRIDINES POUR LE TRAITEMENT DE LA MALADIE D'ALZHEIMER**
1-PHENYLALKYL-1,2,3,6-TETRAHYDROPYRIDINE ZUR BEHANDLUNG VON ALZHEIMERKRANKHEIT
1-PHENYLALKYL-1,2,3,6-TETRAHYDROPYRIDINES FOR TREATING ALZHEIMER'S DISEASE

(30) Priorité: 13.12.1996 FR 9615335
(43) Date de publication de la demande: 20.10.1999
(73) Titulaire: SANOFI-SYNTHELABO, 75013 Paris (FR)
(72) Inventeur: BARONI, Marco, I-20010 Vanzago (IT); CARDAMONE, Rosanna, I-22100 Como (IT); FOURNIER, Jacqueline, F-31830 Plaisance du Touch (FR); GUZZI, Umberto, I-20148 Milan (IT)
(74) Mandataire: Gillard, Marie-Louise
(86) Numéro de dépôt international: FR9702286
(87) Numéro de publication internationale: WO9825903

(56) Documents cités:
- EP-A- 0 412 901
- EP-A- 0 458 696
- WO-A-93/11107
- FR-A- 2 070 102

## Description

La présente invention concerne de nouvelles 1-phénylalkyl-1,2,3,6-tétrahydropyridines 4-substituées ayant une activité neurotrophique et neuroprotectrice, un procédé pour leur préparation, et des compositions pharmaceutiques les contenant

EP-0 458 696 décrit l'utilisation d'une 1-(2-naphtyléthyl)-4-(3-trifluorométhylphényl)-1,2,3,6-tétrahydropyridine pour la préparation de médicaments destinés au traitement de troubles cérébraux et neuronaux.

WO 93/11107 décrit des pipéridines et tétrahydropyridines à activité protectrice vis-à-vis des dommages causés par les états hypoxiques/ischémiques.

Il a été maintenant trouvé que certaines phénylalkyl-1,2,3,6-tétrahydropyridines, substituées par un groupe phényle ou pyridyle, exercent une action neurotrophique sur le système nerveux semblable à celle du facteur de croissance nerveuse (NGF de l'anglais Nerve Growth Factor) et peuvent rétablir le fonctionnement des cellules endommagées ou présentant des anomalies dans leurs fonctions physiologiques.

La présente invention concerne donc, selon un de ses aspects, les composés de formule (I) 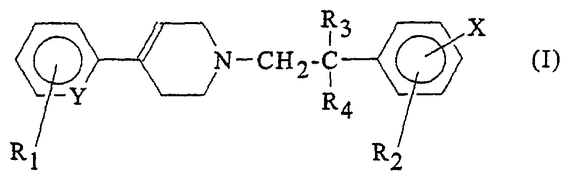 dans laquelle
- Y: représente -CH- ou -N-;
- R₁: représente l'hydrogène, un halogène, un groupe CF₃, (C₁-C₄)alkyle ou (C₁-C₄)alcoxyle;
- R₂: représente un groupe méthyle ou éthyle;
- R₃ et R₄: représentent chacun l'hydrogène ou un (C₁-C₃)alkyle;
- X: représente
(a) un (C₁-C₆)alkyle; un (C₁-C₆)alcoxyle; un (C₃-C₇)carboxyalkyle; un (C₁-C₄)alcoxycarbonyl(C₁-C₆)alkyle; un (C₃-C₇)carboxyalcoxyle; ou un (C₁-C₄)alcoxycarbonyl(C₁-C₆)alcoxyle;
(b) un radical choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit radical pouvant être substitué par un halogène, hydroxy, (C₁-C₄)alcoxy, carboxy, (C₁-C₄)alcoxycarbonyle, amino, mono- ou di-(C₁-C₄)alkylamino; ou
(c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, CF₃, (C₁-C₄)alkyle, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou di-(C₁-C₄)alkylaminocarbonyle, amino(C₁-C₄)alkyle, hydroxy(C₁-C₄)alkyle ou halogéno(C₁-C₄)alkyle;
ainsi que leurs sels et solvates et leurs sels d'ammonium quaternaire.

Dans la présente description le terme "(C₁-C₃)alkyle" désigne les groupes méthyle, éthyle, *n*-propyle et *i*-propyle.

Le terme "(C₁-C₄)alkyle" désigne les groupes méthyle, éthyle, *n*-propyle, *i*-propyle, *n*-butyle, *i*-butyle, *s*-butyle et *t*-butyle.

Le terme "(C₁-C₆)alkyle" désigne un radical hydrocarbonné, contenant de 1 à 6 atomes de carbone tel que, par exemple, méthyle, éthyle, *n*-propyle, *i*-propyle, *n*-butyle, *i*-butyle, *s*-butyle, *t*-butyle, *n*-pentyle, *i*-pentyle, neopentyle, *t*-pentyle, *n*-hexyle, *i*-hexyle, etc.

Le terme "alcoxy" désigne un groupe hydroxyle substitué par un groupe (C₁-C₆)alkyle, avantageusement (C₁-C₄)alkyle, de préférence (C₁-C₃)alkyle.

Lorsque X représente un groupe phényle, la nomenclature donnée au radical biphénylyle est celle conforme aux règles IUPAC, à savoir la numérotation des positions des deux cycles est la suivante: 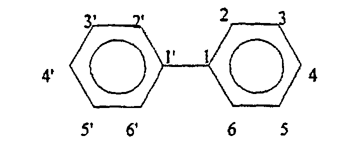 et les radicaux ayant cette structure sont nommés

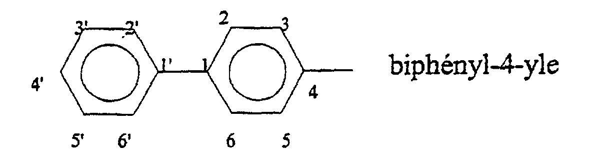

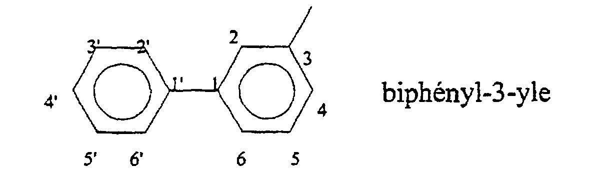

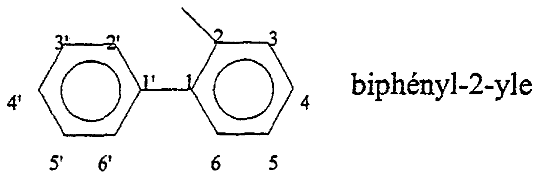

Parmi les composés de formule (I) où X est un groupe (c), un groupe préféré est représenté par les composés où le phényle est substitué par 1 à 3 halogènes, 1 à 3 CF₃, 1 à 3 (C₁-C₄)alkyle, 1 à 3 (C₁-C₄)alcoxy, 1 à 3 cyano, 1 à 3 amino, 1 à 3 mono- ou di-(C₁-C₄)alkylamino, 1 à 3 (C₁-C₄)acylamino, 1 à 3 carboxy, 1 à 3 (C₁-C₄)alcoxycarbonyle, 1 à 3 aminocarbonyle, 1 à 3 mono- ou di-(C₁-C₄)alkylaminocarbonyle, 1 à 3 amino(C₁-C₄)alkyle, 1 à 3 hydroxy(C₁-C₄)alkyle ou 1 à 3 halogéno(C₁-C₄)alkyle.

Un autre groupe préféré est constitué par les composés de formule (I) où Y est un groupe -CH- et R₁ est CF₃.

Un autre groupe préféré est constitué par les composés de formule (I) où Y est un atome d'azote et R₁ est un atome de chlore.

Un autre groupe préféré est constitué par les composés de formule (I) où X est un groupe (C₁-C₆)alkyle, notamment éthyle.
Des composés particulièrement avantageux sont représentés par la formule (I') 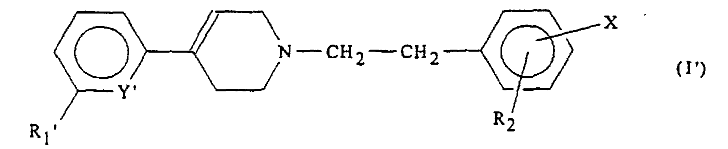 dans laquelle R₁' est CF₃ et Y ' est CH ou bien R₁' est Cl et Y' est N, R₂ et X étant tels que définis ci-dessus et leurs sels, solvates et sels d'ammonium quaternaire.

Un autre groupe préféré est constitué par les composés de formule (I') où X est un groupe (C₁-C₆)alkyle.

Des composés particulièrement avantageux selon la présente invention sont les suivants:
la 1-[2-(3,4-diéthylphényl)éthyl]-4-(3-trifluorométhylphényl)-l,2,3,6-tétrahydropyridine, la 1-[2-(3-méthyl-4-pentylphényl)éthyl)-4-(3-trifluorométhylphényl)-1,2,3,6-tétrahydropyridine, la 1 -[2-(4-méthyl-3-pentylphényl)éthyl]-4-(3-trifluorométhylphényl)-1,2,3,6-tétrahydropyridine, la 1-[2-(3,4-diéthylphényl)éthyl]-4-(6-chloropyrid-2-yl)-1,2,3,6-tétrahydropyridine et leurs sels, solvates et sels d'ammonium quaternaire.

Selon un autre de ses aspects, la présente invention concerne un procédé pour la préparation des composés de formule (I), de leurs sels ou solvates et de leurs sels d'ammonium quaternaire, caractérisé en ce que
(a) on fait réagir une aryl-1,2,3,6-tétrahydropyridine de formule (II) 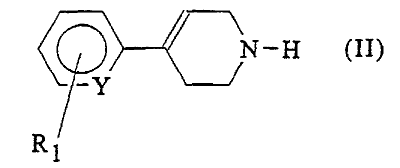 dans laquelle Y et R₁ sont tels que définis ci-dessus, avec un composé de formule (III) 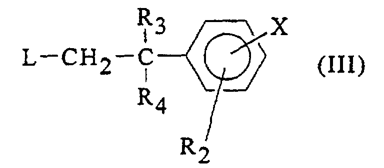 dans laquelle R₂, R₃, R₄ et X sont tels que définis précédemment et L représente un groupe partant, tel que par exemple un atome de chlore, de brome, d'iode, le groupe méthanesulfonyloxy, p-toluènesulfonyloxy, trifluorométhanesulfonyloxy; et
(b) on isole le composé de formule (I) ainsi obtenu et, éventuellement, on le transforme en l'un de ses sels ou solvates ou l'un de ses sels d'ammonium quaternaire.

La réaction est conduite dans un solvant organique et à une température comprise entre la température ambiante et la température de reflux du solvant utilisé.

Comme solvant organique préférentiel, on utilise un alcool aliphatique ayant de 1 à 6 atomes de carbone, tel que méthanol, éthanol, isopropanol, n-butanol, n-pentanol, mais d'autres solvants tels que hexane, diméthylformamide, diméthylsulfoxyde, sulfolane, acétonitrile, pyridine et similaires peuvent être également utilisés.

La réaction est avantageusement conduite en présence d'un agent basique, tel qu'un carbonate alcalin ou la triéthylamine, surtout lorsque L est un atome d'halogène.

La température de réaction peut varier entre la température ambiante (environ 20°C) et celle de reflux et les temps de réaction varient en conséquence. En général, après 6 à 12 heures de chauffage au reflux, la réaction est terminée et le produit final ainsi obtenu peut être isolé selon les techniques conventionnelles sous forme de base libre ou d'un de ses sels, la base libre étant éventuellement transformée en l'un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu est isolé selon les techniques usuelles et éventuellement transformé en l'un de ses sels d'addition d'acides ou, lorsqu'un groupe acide est présent, le caractère amphotère du composé permet la séparation des sels soit avec des acides soit avec des bases.

Lorsque les sels du composé de formule (I) sont préparés pour être administrés en tant que médicaments, il faut que les acides ou les bases employées soient pharmaceutiquement acceptables; si l'on prépare des sels du composé de formule (I) dans un autre but, par exemple pour mieux purifier le produit ou pour mieux effectuer des essais analytiques, on peut alors utiliser n'importe quel acide ou base.

Les sels avec des acides pharmaceutiquement acceptables sont par exemple, ceux avec les acides minéraux, tels que le chlorhydrate, le bromhydrate, le borate, le phosphate, le sulfate, l'hydrogénosulfate, l'hydrogénophosphate, le dihydrogénophosphate et ceux avec les acides organiques, tels que le citrate, le benzoate, l'ascorbate, le méthylsulfate, le naphtalène-2-sulfonate, le picrate, le fumarate, le maléate, le malonate, l'oxalate, le succinate, l'acétate, le tartrate, le mésylate, le tosylate, l'iséthionate, l'α-cétoglutarate, l'α-glycérophosphate, le glucose-1-phosphate, etc.

Les sels avec des bases pharmaceutiquement acceptables des composés de formule (I) dans laquelle le substituant X contient un carboxyle, sont par exemple ceux avec les métaux alcalins ou alcalino-terreux, tels que le sodium, le potassium, le calcium, le magnésium et ceux avec les bases organiques, telles que les amines, les acides aminés basiques (la lysine, l'arginine, l'histidine), le trométamol, la N-méthylglucamine, etc.

Les amines de départ de formule (II) où Y est CH sont des composés connus ou bien elles peuvent être préparées selon des procédés analogues à ceux utilisés pour préparer les composés connus.

Les amines de départ de formule (II) où Y est N peuvent être préparées par réaction de la 2-halogénopyridine appropriée de formule (p) 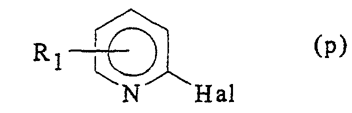 dans laquelle R₁ est tel que défini précédemment et Hal est un atome d'halogène, avec une 1,2,3,6-tétrahydropyridine de formule (q) 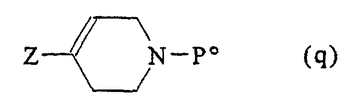 dans laquelle P° représente un groupe protecteur tel que par exemple le groupe benzyle et Z représente un substituant qui permet la substitution nucléophile de l'halogène de la pyridine. De tels substituants sont par exemple les trialkylstannanes, comme le tributylstannane ou les composés de Grignard.

On déprotège ensuite la 1,2,3,6-tétrahydropyridine par clivage du groupe protecteur dans des conditions convenables.

Les composés de formule (III) peuvent être préparés
- soit, pour préparer un composé de formule (III) dans laquelle R₃ = R₄ = H, par réaction du benzène approprié de formule (r) 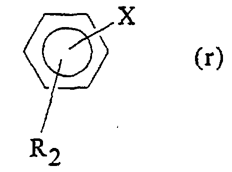 dans laquelle R₂ et X sont tels que définis précédemment, avec un halogénure d'acyle de formule L-CH₂-CO-Hal dans laquelle L et Hal sont tels que définis précédemment, en présence d'un acide de Lewis selon la réaction bien connue de Friedel-Crafts, et par réduction de la cétone de formule (s) ainsi obtenue 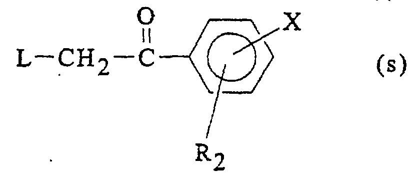 selon les méthodes opératoires largement décrites en littérature;
- soit par réduction des acides de formule (V) 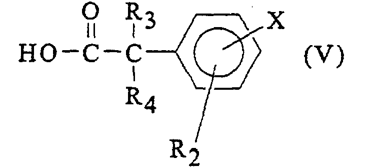 où R₂, R₃, R₄ et X sont tels que définis précédemment, en alcool, suivie par la transformation de la fonction hydroxyle en groupe partant.

Les acides de formule (V) sont en général des composés décrits en littérature ou bien ils peuvent être préparés de façon analogue.

Des exemples de préparation sont également consignés dans la partie expérimentale.

L'activité des composés de formule (I) sur le système nerveux a été démontrée dans des études *in vitro* et *in vivo* selon les méthodes décrites dans EP-0 458 696 et, pour l'évaluation de la survie neuronale, à l'aide d'un test de survie *in vitro* conduit en utilisant des neurones isolés à partir de dissections de la région septale d'embryons de rats.

Selon ce test, on préleve la région septale d'embryons de rats agés de 17-18 jours sous microscope à dissection dans des conditions stériles, puis on la dissocie dans un milieu trypsine-EDTA. La suspension de cellules est placée dans un flacon de culture dans un milieu DME/Ham's F12 (v:v) (Dulbecco Modified Eagle Medium/ Nutrient Mixture Ham's F12 - R.G. Ham, Proc. Nat. Sci, 1965, 53:288) contenant du sérum de veau à 5% et du sérum de cheval à 5% et maintenue à 37°C pendant 90 minutes. Ce traitement permet l'élimination des cellules non-neuronales.

Les neuroblastes sont ensuite ensemencés dans les puits d'une plaque de titration à raison de 17x10⁴ cellules/cm², dans un milieu de culture non sérique constitué par du DME/Ham's F12 contenant du sélénium (30 nM) et de la transferrine (1,25 µM). Chaque puits a été préalablement traité à la poly-L-lysine. Les plaques ensemencées sont placées dans un incubateur à l'étuve (37°C; 5% CO₂).

Les composés à tester sont dissous dans du DMSO et dilués comme requis par le milieu de culture.

Les neuroblastes sont maintenus dans des plaques contenant le composé à tester ou le solvant correspondant pendant 4 jours sans changer le milieu.

Après 4 jours le milieu est remplacé par un sel de tétrazolium dissous dans le milieu de culture (0,15 mg/ml). Les cellules sont ensuite placées à l'étuve à 37°C pendant 4 heures. Les succinodéshydrogénases mitochondriales des cellules vivantes réduisent le sel de tétrazolium en bleu formazan dont, après dissolution dans le DMSO, on mesure la densité optique à 540 nm. Cette densité est linéairement corrélée au nombre de cellules vivantes (Manthorpe et al., Dev. Brain Res., 1988, 25:191-198).

La différence entre les groupes contenant les composés à tester et les témoins a été évaluée par analyse statistique en utilisant le test t bilatéral de Dunnett ("two-tailed Dunnett t-test").

Dans ce dernier test les composés de formule (I) se sont montrés aussi actifs ou plus actifs que les composés décrits dans EP-0 458 696, l'efficacité de certains composés de formule (I) vis-à-vis de la survie neuronale étant double par rapport au composé A décrit dans EP-0 458 696.

Grâce à cette puissante activité neuroprotectrice, et à leur faible toxicité compatible pour une utilisation en tant que médicaments, les composés de formule (I) ainsi que leurs sels d'addition pharmaceutiquement acceptables, leurs solvates et leurs sels d'ammonium quaternaire sont utilisables pour la préparation de compositions pharmaceutiques indiquées dans le traitement et/ou la prophylaxie de toutes les maladies qui impliquent une dégénérescence neuronale. Plus particulièrement, les composés de l'invention sont utilisables, seuls ou en co-administration ou association avec d'autres principes actifs agissant sur le SNC, par exemple les inhibiteurs de l'acétylcholinestérase, les cholinomimétiques M1 sélectifs, les antagonistes NMDA, les nootropiques tels que le piracétam, notamment dans les indications suivantes: troubles de la mémoire, démence vasculaire, troubles post-encéphalitiques, troubles post-apoplectiques, syndromes post-traumatiques dus à un traumatisme crânien, troubles dérivant d'anoxies cérébrales, maladie d'Alzheimer, démence sénile, démence subcorticale, telle que la chorée de Huntington et la maladie de Parkinson, démence provoquée par le SIDA, neuropathies dérivées de morbidité ou dommage des nerfs sympathiques ou sensoriels, et maladies cérébrales, telles que l'oedème cérébral, et les dégénérescences spinocérébelleuses, les dégénérescences des motoneurones, comme par exemple la sclérose latérale amyotrophique.

L'administration des composés de l'invention peut être convenablement effectuée par voie orale, parentérale, sublinguale ou transdermique. La quantité de principe actif à administrer dans le traitement des troubles cérébraux et neuronaux selon la méthode de la présente invention dépend de la nature et de la gravité des affections à traiter ainsi que du poids des malades. Néanmoins, les doses unitaires préférées comprendront généralement de 0,25 à 700 mg, avantageusement de 0,5 à 300 mg, de préférence de 1 à 150 mg, par exemple entre 2 et 50 mg, à savoir 2, 5, 10, 15, 20, 25 ou 50 mg de produit. Ces doses unitaires seront administrées normalement une ou plusieurs fois par jour, par exemple 2, 3, 4, ou 5 fois par jour, de préférence une à trois fois par jour, la dose globale chez l'homme étant variable entre 0,5 et 1400 mg par jour, avantageusement entre 1 et 900 mg par jour, par exemple de 2 à 500 mg, plus convenablement de 2 à 200 mg par jour.

Selon un autre de ses aspects, la présente invention a pour objet une composition pharmaceutique contenant, en tant que principes actifs, un composé de formule (I) ci-dessus et un composé indiqué dans le traitement symptomatique de la démence sénile du type Alzheimer (DAT) ou leurs sels pharmaceutiquement acceptables.

L'expression "composé indiqué dans le traitement symptomatique de la démence sénile du type Alzheimer (DAT)" indique un produit qui est capable d'améliorer le cadre symptomatologique des patients atteints par la DAT sans intervenir sur les causes de la maladie.

De tels composés sont par exemple les inhibiteurs de l'acétylcholinestérase, les agonistes muscariniques M₁, les agonistes nicotiniques, les antagonistes du récepteur NMDA, les nootropiques.

Des inhibiteurs de l'acétylcholinestérase préférés sont le donépézil et la tacrine.

D'autres inhibiteurs de l'acétylcholinestérase pouvant être utilisés sont par exemple la rivastigmine (SDZ-ENA-713), la galanthamine, le métrifonate, l'eptastigmine, la velnacrine, la physostigmine (Drugs, 1997, 53(5): 752-768; The Merck Index 12 ed.).

D'autres inhibiteurs de l'acétylcholinestérase sont encore la 5,7-dihydro-3-[2-[1-(phénylméthyl)-4-pipéridinyl]éthyl]-6H-pyrrolo[3,2-f]-1,2-benzisoxazol-6-one nommée aussi icopézil (J. Med. Chem., 1995, 38: 2802-2808), le MDL-73,745 ou zifrosilone (Eur. J. Pharmacol., 1995, 276: 93-99), le TAK-147 (J. Med. Chem., 1994, 37: 2292-2299).

D'autres inhibiteurs de l'acétylcholinestérase sont par exemple ceux qui sont décrits dans les demandes de brevet JP 09-095483, WO 97/13754, WO 97/21681, WO 97119929, ZA 96-04565, US 5,455,245, WO 95-21822, EP 637 586, US 5,401,749, EP 742 207, US 5,547,960, WO 96/20176, WO 96/02524, EP 677 516, JP 07-188177, JP 07-133274, EP 649 846, EP 648 771, JP 07-048370, US 5,391,553, WO 94/29272, EP 627 400.

Selon un autre de ses aspects, la présente invention concerne une composition pharmaceutique contenant, en tant que principes actifs, un composé de formule (I) et un agoniste du récepteur M₁, ou leurs sels pharmaceutiquement acceptables.

Des agonistes du récepteur M₁ sont par exemple la milaméline, la bésipiridine, la talsaclidine, la xanoméline, le YM-796 et le YM-954 (Eur. J. Pharmacol., 1990, 187: 479-486), la 3-[N-(2-diéthylamino-2-méthylpropyl)-6-phényl-5-propyl]pyridazinamine, nommée aussi SR-46559 (Biorg. Med. Chem. Let., 1992, 2: 833:838), le AF-102, le CI-979, le L-689,660, le LU 25-109, le S-9977-2, le SB 202,026, la thiopilocarpine, le WAL 2014 (Pharmacol. Toxicol., 1996, 78: 59-68).

Selon un autre de ses aspects, l'invention concerne une composition pharmaceutique contenant, en tant que principes actifs, un composé de formule (I) et un agoniste nicotinique ou leurs sels pharmaceutiquement acceptables.

Des agonistes nicotiniques avantageux sont par exemple le MKC-231 (Biorg. Med. Chem. Let., 1995, 5 (14): 1495-1500), le T-588 (Japan J. Pharmacol., 1993, 62: 81-86), le ABT-418 (Br. J. Pharmacol., 1997, 120: 429-438).

Selon un autre de ses aspects, l'invention concerne une composition pharmaceutique contenant, en tant que principes actifs, un composé de formule (I) et un antagoniste des récepteurs NMDA ou leurs sels pharmaceutiquement acceptables.

Un antagoniste des récepteurs NMDA avantageux est par exemple la mémantine (Arzneim. Forsch., 1991, 41: 773-780).

Selon un autre de ses aspects, l'invention concerne une composition pharmaceutique contenant, en tant que principes actifs un composé de formule (I) et un agent nootropique, ou leurs sels pharmaceutiquement acceptables.

Des agents nootropiques qui peuvent être utilisés selon l'invention sont par exemple le nétiracétam, le nébracétam (Merck Index, 12^{th} ed.).

Les doses des deux principes actifs associés sont choisies en général parmi les doses qui seraient administrées pour chaque médicament dans le traitement non combiné.

Selon un aspect ultérieur, la présente invention concerne aussi une méthode de traitement de la démence sénile du type Alzheimer qui consiste à administrer à un patient atteint de cette maladie une dose efficace d'un composé de formule (I) ou d'un de ses sels pharmaceutiquement acceptables et une dose efficace d'un composé indiqué dans le traitement symptomatique de la DAT ou d'un de ses sels pharmaceutiquement acceptables, lesdites administrations étant simultanées, séquentielles ou étalées dans le temps et les doses efficaces des principes actifs pouvant être contenues dans des formes d'administration unitaires séparées ou bien, lorsque les principes actifs sont administrés simultanément, les deux principes actifs sont avantageusement contenus dans une forme pharmaceutique unique.

Dans les compositions pharmaceutiques de la présente invention pour l'administration orale, sublinguale, sous-cutanée, intramusculaire, intraveineuse, transdermique ou rectale, le principe actif peut être administré sous formes unitaires d'administration, soit tel quel par exemple sous forme lyophilisée, soit en mélange avec des supports pharmaceutiques classiques, aux animaux et aux êtres humains pour le traitement des affections susdites. Les formes unitaires d'administration appropriées comprennent les formes par voie orale telles que les comprimés éventuellement sécables, les gélules, les poudres, les granules et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Les exemples qui suivent illustrent mieux l'invention.

### EXEMPLE 1

### Chlorhydrate de 1-[2-(3,4-diéthylphényl)éthyl]-4-(3-trifluorométhylphényl)-1,2,3,6-tétrahydropyridine.

**1a/ *1-bromo-2-(3,4-diéthylphényl)éthane.***
   On refroidit à 0-5°C un mélange de 4,4 g (0,033 mole) de 3,4-diéthylbenzène, 50 ml de chlorure de méthylène, 8,8 g (0,044 mole) de bromure de bromoacétyle et on y ajoute 5,0 g (0,037 mole) de trichlorure d'aluminium. On agite à 0-5°C pendant une heure puis on laisse une nuit à la température ambiante. On verse dans un mélange eau/glace, on extrait au chlorure de méthylène, on sèche la phase organique sur du sulfate de sodium et on évapore le solvant sous pression réduite. On mélange 2,9 g (0,011 mole) de l'huile ainsi obtenue avec 6 ml (0,079 mole) d'acide trifluoroacétique et 6,7 ml (0,057 mole) de triéthylsilane et on chauffe à 80°C pendant 4 heures. On ajoute ensuite une solution aqueuse saturée de bicarbonate de sodium jusqu'à pH basique, on extrait à l'éther éthylique, on sèche la phase organique sur du sulfate de sodium et on évapore le solvant sous pression réduite. On purifie l'huile brute ainsi obtenue par chromatographie sur colonne de gel de silice en éluant avec du cyclohexane. On obtient le composé du titre.
**1b/ *Chlorhydrate de 1-[2-(3,4-diéthylphényl)éthyl]-4-(3-trifluorométhylphényl)-1,2,3,6-tetrahydropyridine.***
   On chauffe au reflux pendant 5 heures un mélange de 2,6 g (0,001 mole) de 4-(3-trifluorométhylphényl)-1,2,3,6-tétrahydropyridine, 60 ml de butanol, 4,1 g (0,025 mole) de carbonate de potassium anhydre râpé et 2,6 g (0,00113 mole) du produit de l'étape précédente. On évapore le solvant sous pression réduite, on reprend à l'acétate d'éthyle, on lave à l'eau, on sèche sur du sulfate de sodium et on évapore le solvant sous pression réduite. On prépare le chlorhydrate de l'huile ainsi obtenue par traitement avec une solution d'isopropanol saturée en acide chlorhydrique. On obtient 1,6 g du composé du titre. P.f. 220-222°C.

### EXEMPLE 2

### 1-[2-(3-méthyl-4-pentylphényl)éthyl]-4-(3-trifluorométhylphényl)-1,2,3,6-tétrahydropyridine et 1-[2-(4-méthyl-3-pentylphényl)éthyl]-4-(3-trifluorométhylphényl)-1,2,3,6-tétrahydropyridine et leurs oxalates.

**2a/ *1-méthyl-2-pentylbenzène.***
   A une solution de 50 ml (0,1 mole) d'une solution de chlorure de n-butylmagnésium 2M dans le THF sous atmosphère d'azote, on ajoute goutte à goutte 4,7 g (0,035 mole) d'aldéhyde phtalique. Le mélange se chauffe spontanément à 40-45°C. On agite à la température ambiante pendant une heure, on verse dans une solution saturée de chlorure d'ammonium. On extrait à l'éther éthylique, on lave à l'eau, on sèche sur du sulfate de sodium et on évapore le solvant sous pression réduite. On purifie l'huile ainsi obtenue par chromatographie sur colonne de gel de silice en éluant par un mélange cyclohexane/acétate d'éthyle = 7/3. On isole le produit ayant le Rf le plus élevé. On obtient 2,0 g d'huile. On dissout le brut de réaction dans 25 ml d'éthanol et on y ajoute 1 ml d'acide sulfurique concentré et 0,15 g de Pd/C à 10%. On hydrogène à la température ambiante pendant 7 heures. On filtre le catalyseur, on évapore le solvant sous pression réduite et on reprend le résidu à l'acétate d'éthyle. On lave avec une solution aqueuse de bicarbonate de sodium, on sèche et on évapore le solvant sous pression réduite. On obtient 1,35 g du produit du titre.
**2b/ *1-bromo-2-(3-méthyl-4-pentylphényl)éthane et 1-bromo-2-(4-méthyl-3-pentylphényl)éthane.***
   On refroidit à 0-5°C un mélange de 1,17 g (0,0054 mole) du produit de l'étape précédente, 0,62 ml (0,0072 mole) de bromure de bromoacétyle et on y ajoute 0,81 g (0,006 mole) de trichlorure d'aluminium. On agite à 0-5°C pendant une heure et ensuite 4 heures à la température ambiante. On verse dans de la glace, on sépare les deux phases, on lave la phase organique à l'eau, on la sèche et on évapore le solvant sous pression réduite. On dissout le résidu dans 2,9 ml d'acide trifluoroacétique et on y ajoute 3,1 ml (0,0267 mole) de triéthylsilane et on chauffe le mélange à 80°C pendant 5 heures. On verse dans une solution aqueuse de bicarbonate de sodium et on extrait à l'éther éthylique. On lave à l'eau, on sèche sur du sulfate de sodium. On obtient un mélange des composés du titre.
***2c/ 1-[2-(3-méthyl-4-pentylphényl)éthyl]-4-(3-trifluorométhylphényl)-1,2,3,6-tétrahydropyridine et 1-[2-(4-méthyl-3-pentylphényl)éthyl]-4-(3-trifluorométhylphényl)-1,2,3,6-tétrahydropyridine et leurs oxalates.***
   On chauffe au reflux pendant 6 heures un mélange de 0,7 g (0,0031 mole) de 4-(3-trifluorométhylphényl)-1,2,3,6-tétrahydropyridine, 16 ml de butanol, 0,9 g (0,0065 mole) de carbonate de potassium anhydre râpé et le produit obtenu à l'étape précédente (0,0054 mole théorique). On évapore le solvant sous pression réduite, on reprend à l'acétate d'éthyle, on lave à l'eau, on sèche sur du sulfate de sodium et on évapore le solvant sous pression réduite. On purifie l'huile ainsi obtenue par chromatographie sur colonne de gel de silice en éluant par un mélange cyclohexane/acétate d'éthyle = 7/3. On isole deux produits ayant un Rf similaire. Le produit ayant le Rf le plus élevé correspond à la 1-[2-(3-méthyl-4-pentylphényl)éthyl]-4-(3-trifluorométhylphényl)-1,2,3,6-tétrahydropyridine. On prépare l'oxalate dans de l'acétone. On obtient 0,12 g de produit. P.f. 140-143°C. Le produit ayant le Rf le plus faible correspond à l'isomère 1-[2-(4-méthyl-3-pentylphényl)éthyl]-4-(3-trifluorométhylphényl)-1,2,3,6-tétrahydropyridine. On prépare l'oxalate dans de l'acétone. On cristallise le produit dans de l'acétone. On obtient 0,08 g de produit. P.f. 167-169°C.

### EXEMPLE 3

### Chlorhydrate de 1-[2-(3,4-diéthylphényl)éthyl]-4-(6-chloropyrid-2-yl)-1,2,3,6-tétrahydropyridine.

**3a/ *(1-benzyl-1,2,3,6-tétrahydropyrid-4-yl)tributylstannane.***
   On agite à la température ambiante pendant 3 heures un mélange de 15,85 g (0,0837 mole) de 1-benzyl-4-pipéridone dans 140 ml de diméthoxyéthane anhydre et 25 g (0,0837 mole) de trisilidrazine dans 140 ml de diméthoxyéthane anhydre. On évapore le solvant sous pression réduite. On reprend le résidu dans 420 ml d'hexane anhydre et on y ajoute 420 ml de tétraméthyléthylènediamine anhydre. On refroidit le mélange à -78°C et on y ajoute goutte à goutte 156 ml de n-butyl lithium (0,25 mole) (solution 1,6 M dans l'hexane). Après environ 30 minutes on laisse revenir la température jusqu'à 0°C et on agite pendant 15 minutes. On ajoute ensuite au mélange réactionnel 45 ml (0,167 mole) de chlorure de tributylstannane. Après 1 heure on ajoute, avec extrême précaution un mélange eau/glace. On extrait à l'éther éthylique, on lave la phase organique à l'eau, on sèche sur du sulfate de sodium et on évapore le solvant sous pression réduite. On obtient 70 g de produit brut qu'on purifie par chromatographie sur colonne de gel de silice en éluant avec un mélange cyclohexane/acétate d'éthyle = 95/5. On obtient le composé du titre sous forme d'huile.
   ¹H-RMN (CDCl₃) - d (ppm) : 0,84 (9H; m: CH₃); 1,19-1,58 (18H; m: CH₂ - chaîne); 2,31 (2H; m); 2,53 (2H; m); 3,02 (2H; m); 3,56 (2H; s: méthylène benzylique); 5,76 (1H; m*); 7,18-7,41 (5H; m: arom.)
   * bandes satellites ³j_{cis}(¹H-¹¹⁷Sn) et ³J_{cis}(¹H-¹¹⁹Sn).
**3b/ *1-benzyl-4-(6-chloropyrid-2-yl)-1,2,3,6-tétrahydropyridine.***
   On dissout 18,5 g (0,04 mole) du composé de l'étape précédente dans 200 ml de diméthylformamide anhydre sous atmosphère d'azote. On ajoute à la solution 11,8 g (0,08 mole) de 2,6-dichloropyridine, 0,64 g de Pd(II) (Ph₃P)₂Cl₂, 4,38 g (0,04 mole) de chlorure de tétraméthylammonium et 2,76 g (0,02 mole) de carbonate de potassium. On chauffe à 110°C pendant 6 heures puis on verse le mélange dans 100 ml d'une solution d'acide sulfurique à 5%. On extrait à l'éther éthylique, on ajoute de l'hydroxyde d'ammonium à la phase aqueuse juqu'à pH basique et on extrait à l'acétate d'éthyle. On sèche les phases organiques réunies sur du sulfate de sodium et on évapore le solvant sous pression réduite. On purifie le résidu par chromatographie sur colonne de gel de silice en éluant avec un mélange cyclohexane/acétate d'éthyle = 1/1. On obtient le composé du titre. P.f. 100-102°C.
**3c/ *Chlorhydrate de 4*-*(6-chloropyrid*-*2*-*yl)-1,2,3,6-tétrahydropyridine***.
   On refroidit à 0-5°C une solution de 7,0 g (0,024 mole) du composé de l'étape précédente dans 110 ml de dichloroéthane et on y ajoute 5,8 ml (0,054 mole) de chloroformiate de chloroéthyle. On agite pendant 5 minutes puis on chauffe au reflux pendant 1,5 heures. On évapore le solvant sous pression réduite, on reprend le résidu dans 100 ml de méthanol et on chauffe au reflux pendant 1 heure. On évapore le solvant, on reprend le résidu dans de l'isopropanol et on filtre le solide. On obtient le composé du titre qu'on cristallise dans de l'éthanol à 90%. P.f. 305-307°C.
**3d/ *Chlorhydrate de 1-[2-(3,4-diéthylphényl)éthyl]-4-(6-chloropyrid-2-yl)-1,2,3,6-tétrahydropyridine.***
   En opérant comme décrit dans l'exemple 1b/ mais en utilisant le produit de l'étape précédente au lieu de la 4-(3-trifluorométhylphényl)-1,2,3,6-tétrahydropyridine, on obtient le composé du titre. P.f. 234-236°C.

### EXEMPLES 4-13

En opérant comme décrit dans l'exemple 2 mais en utilisant l'halogénure de magnésium approprié, on obtient les composés suivants:
**1-[2-(3-éthyl-4-méthylphényl)éthyl]-4-(3-trifluorométhylphényl)-1,2,3,6-tétrahydropyridine - Ex. 4**
**1-[2-(4-éthyl-3-méthylphényl)éthyl]-4-(3-trifluorométhylphényl)-1,2,3,6-tétrahydropyridine - Ex. 5**
**1-[2-(3-éthyl-4-propylphényl)éthyl]-4-(3-trifluorométhylphényl)-1,2,3,6-tétrahydropyridine - Ex. 6**
**1-[2-(4-éthyl-3-propylphényl)éthyl]-4-(3-trifluorométhylphényl)-1,2,3,6-tétrahydropyridine - Ex. 7**
**1-[2-(3-butyl-4-méthylphényl)éthyl]-4-(3-trifluorométhylphényl)-1,2,3,6-tétrahydropyridine - Ex. 8**
**1-[2-(4-butyl-3-méthylphényl)éthyl]-4-(3-trifluorométhylphényl)-1,2,3,6-tétrahydropyridine - Ex.9**
**1-[2-(3-*iso*butyl-4-méthylphényl)éthyl]-4-(3-trifluorométhylphényl)-1,2,3,6-tétrahydropyridine - Ex. 10**
**1-[2-(4-*iso*butyl-3-méthylphényl)éthyl]-4-(3-trifluorométhylphényl)-1,2,3,6-tétrahydropyridine - Ex. 11**
**1-[2-(3-*iso*butyl-4-éthylphényl)éthyl]-4-(3-trifluorométhylphényl)-1,2,3,6-tétrahydropyridine - Ex.12**
**1-[2-(4-*iso*butyl-3-éthylphényl)éthyl]-4-(3-trifluorométhylphényl)-1,2,3,6-tétrahydropyridine - Ex. 13**

### EXEMPLE 14

### 1-[2-(6-méthyl-3-biphénylyl)éthyl]-4-(3-trifluorométhylphényl)-1,2,3,6-tétrahydropyridine

En opérant comme décrit dans l'exemple 2 mais en utilisant le phényl-lithium au lieu du chlorure de n-butylmagnésium, on obtient le composé du titre.

### EXEMPLE 15

### Chlorhydrate de 1-[2-(3,4-diméthylphényl)éthyl]-4-(3-trifluorométhylphényl)-1,2,3,6-tétrahydropyridine

**15a/ *1-bromo-2-(3,4-dimethylphényl)éthane***.
   On refroidit à 0-5°C 4,5 g (0,03 mole) de 3,4-diméthylacétophénone dans 12 ml de méthanol et on y ajoute goutte à goutte 1,5 ml (0,09 mole) de brome. On agite à la température ambiante pendant 24 heures puis on laisse une nuit à la température ambiante. On évapore le méthanol et on purifie le mélange par chromatographie sur colonne de gel de silice en éluant avec un mélange cyclohexane/acétate d'éthyle = 95/5. On mélange 5,3 g (0,013 mole) du produit ainsi obtenu avec 12,5 ml (0,162 mole) d'acide trifluoroacétique et 18,7 ml (0,011 mole) de triéthylsilane et on chauffe à 80°C pendant 1 heure. On ajoute ensuite une solution aqueuse saturée de bicarbonate de sodium jusqu'à pH basique, on extrait à l'éther éthylique, on sèche la phase organique sur du sulfate de sodium et on évapore le solvant sous pression réduite. On obtient 5,2 g du composé du titre.
**15b/ *Chlorhydrate de 1-[2-(3,4-diméthylphényl)éthyl]-4-(3-trifluorométhylphényl)-1,2,3,6-tétrahydropyridine.***
   On chauffe au reflux pendant 6 heures un mélange de 1,8 g (0,0068 mole) de 4-(3-trifluorométhylphényl)-1,2,3,6-tétrahydropyridine, 25 ml de butanol, 2,4 g (0,017 mole) de carbonate de potassium anhydre râpé et 2 g (0,0094 mole) du produit de l'étape précédente. On évapore le solvant sous pression réduite, on reprend à l'acétate d'éthyle, on lave à l'eau, on sèche sur du sulfate de sodium et on évapore le solvant sous pression réduite. On purifie le produit par chromatographie sur colonne de gel de silice en éluant avec un mélange cyclohexane/acétate d'éthyle = 7/3. On prépare le chlorhydrate de l'huile ainsi obtenue par traitement avec une solution d'isopropanol saturée en acide chlorhydrique. On obtient 1,1 g du composé du titre. P.f. 270-272°C.

### EXEMPLE 16

### Chlorhydrate de 1-[2-(3,4-diéthylphényl)éthyl]-4-(2-trifluorométhylphényl)-1,2,3,6-tétrahydropyridine

**16a/ *Chlorhydrate de* 4-hydroxy-4-(2-trifluorométhylphényl)pipéridine.**
   On mélange 3,25 g (0,135 mole) de Mg avec une pointe de spatule de I₂ et on y ajoute goutte à goutte une solution de 30,4 g (0,135 mole) de 2-bromo-1-trifluorométhylbenzène dans 125 ml de THF. On agite pendant une heure à la température ambiante et on y ajoute goutte à goutte 10,1 g (0,041 mole) de benzylpipéridone. On agite pendant 1 heure à la température ambiante et on ajoute au mélange une solution saturée en chlorure d'ammonium. On extrait à l'éther éthylique, on sèche la phase organique et on évapore le solvant sous pression réduite. On purifie le produit par chromatographie sur colonne de gel de silice en éluant avec un mélange cyclohexane/acétate d'éthyle. On obtient 6,8 g de 1-benzyl-4-hydroxy-4-(2-trifluorométhylphényl)pipéridine qu'on hydrogène dans 75 ml d'éthanol à 95% à pH acide par ajout d'acide chlorhydrique, à l'aide de 0,7 g de Pd/C à 10 %, en chauffant à la température de 60°C pendant 8 heures. On filtre le catalyseur et on obtient ainsi 2,1 g du produit du titre. P.f. 247-251°C.
**16b/ *Chlorhydrate de 4-(2-trifluorométhylphényl)-1,2,3,6-tétrahydropyridine.***
   On dissout 2,0 g (0,007 mole) du produit de l'étape précédente dans 12 ml d'acide acétique glacial. On y ajoute goutte à goutte 3 ml d'acide sulfurique concentré et on chauffe à 100 °C pendant deux heures. On verse dans de la glace, on ajoute au mélange une solution concentrée de NaOH jusqu'à pH basique et on extrait au chlorure de méthylène. On sèche la phase organique et on évapore le solvant sous pression réduite. On reprend le produit dans 15 ml d'isopropanol et on obtient la 4-(2-trifluorométhylphényl)-1,2,3,6-tétrahydropyridine. On prépare le chlorhydrate à l'aide d'une solution d'acide chlorhydrique dans de l'isopropanol. On obtient 0,9 g du composé du titre. P.f. 213-215°C.
**16c/ *Chlorhydrate de 1-[2-(3,4-diéthylphényl)éthyl]-4-(2-trifluorométhylphényl)-1,2,3,6-tétrahydropyridine.***
   On chauffe au reflux pendant 30 minutes 0,4 g (0,0015 mole) du produit de l'étape précédente et 0,52 g (0,0037 mole) de K₂CO₃ anhydre dans 12 ml de butanol. On y ajoute ensuite 0,41 g (0,0017 mole) du produit obtenu à l'exemple 1a/ et on chauffe au reflux pendant 6 heures. On évapore le solvant, on reprend le résidu à l'acétate d'éthyle, on lave à l'eau, on sèche la phase organique et on évapore le solvant sous pression réduite. On purifie le produit par chromatographie sur colonne de gel de silice en éluant avec un mélange cyclohexane/acétate d'éthyle = 8/2. On obtient ainsi la 1-[2-(3,4-diéthylphényl)éthyl]-4-(2-trifluorométhylphényl)-1,2,3,6-tétrahydropyridine. On prépare le chlorhydrate à l'aide d'une solution d'acide chlorhydrique dans de l'isopropanol. On obtient le composé du titre. P.f. 184- 185°C.

## Revendications

1. Composé de formule (I) 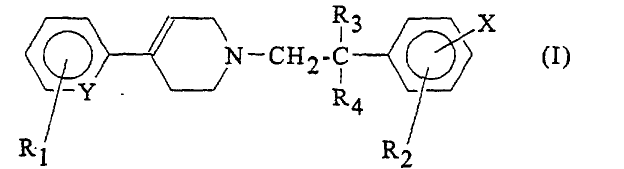 dans laquelle
Y représente -CH- ou -N-;
R₁ représente l'hydrogène, un halogène, un groupe CF₃, (C₁-C₄)alkyle ou (C₁-C₄)alcoxyle;
R₂ représente un groupe méthyle ou éthyle;
R₃ et R₄ représentent chacun l'hydrogène ou un (C₁-C₃)alkyle;
X représente
(a) un (C₁-C₆)alkyle; un (C₁-C₆)alcoxyle; un (C₃-C₇)carboxyalkyle; un (C₁-C₄)alcoxycarbonyl(C₁-C₆)alkyle; un (C₃-C₇)carboxyalcoxyle; ou un (C₁-C₄)alcoxycarbonyl(C₁-C₆)alcoxyle;
(b) un radical choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit radical pouvant être substitué par un halogène, hydroxy, (C₁-C₄)alcoxy, carboxy, (C₁-C₄)alcoxycarbonyle, amino, mono- ou di-(C₁-C₄)alkylamino; ou
(c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, CF₃, (C₁-C₄)alkyIe, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou di-(C₁-C₄)alkylaminocarbonyle, amino(C₁-C₄)alkyle, hydroxy(C₁-C₄)alkyle ou halogéno(C₁-C₄)alkyle;
ainsi que ses sels et solvates et ses sels d'ammonium quaternaire.

2. Composé selon la revendication 1, dans lequel Y est un groupe -CH- et R₁ est CF₃.

3. Composé selon la revendication 1, dans lequel Y est un atome d'azote et R₁ est un atome de chlore.

4. Composé selon l'une des revendications 1 à 3, dans lequel X est un groupe (C₁-C₆)alkyle.

5. Composé selon la revendication 1, dans lequel X est un groupe (c) où le phényle est substitué par 1 à 3 halogènes, 1 à 3 CF₃, 1 à 3 (C₁-C₄)alkyle, 1 à 3 (C₁-C₄)alcoxy, 1 à 3 cyano, 1 à 3 amino, 1 à 3 mono- ou di-(C₁-C₄)alkylamino, 1 à 3 (C₁-C₄)acylamino, 1 à 3 carboxy, 1 à 3 (C₁-C₄)alcoxycarbonyle, 1 à 3 aminocarbonyle, 1 à 3 mono- ou di-(C₁-C₄)alkylaminocarbonyle, 1 à 3 amino(C₁-C₄)alkyle, 1 à 3 hydroxy(C₁-C₄)alkyle ou 1 à 3 halogéno(C₁-C₄)alkyle.

6. Composé selon la revendication 1 de formule (I') 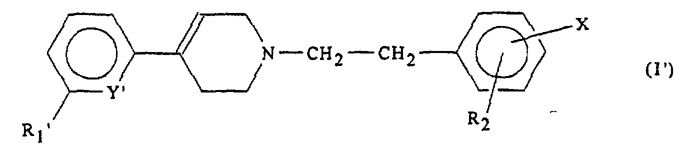 dans laquelle R₁' est CF₃ et Y ' est CH ou bien R₁' est Cl et Y' est N et R₂ et X sont tels que définis pour les composés (I) dans la revendication 1, ainsi que ses sels et solvates et sels d'ammonium quaternaire.

7. Composé selon la revendication 6, dans lequel X est un groupe (C₁-C₆)alkyle.

8. Composé selon la revendication 1, choisi parmi la 1-[2-(3,4-diéthylphényl)éthyl]-4-(3-trifluorométhylphényl)-1,2,3,6-tétrahydropyridine, la 1-[2-(3-méthyl-4-pentylphényl)éthyl]-4-(3-trifluorométhylphényl)-1,2,3,6-tétrahydropyridine, la 1-[2-(4-méthyl-3-pentylphényl)éthyl]-4-(3-trifluorométhylphényl)-1,2,3,6-tétrahydropyridine, la 1-[2-(3,4-diéthylphényl)éthyl]-4-(6-chloropyrid-2-yl)-1,2,3,6-tétrahydropyridine et leurs sels, solvates ou sels d'ammonium quaternaire.

9. Procédé pour la préparation des composés de formule (I) selon la revendication 1, de leurs sels ou solvates et de leurs sels d'ammonium quaternaire, caractérisé en ce que:
(a) on fait réagir une aryl-1,2,3,6-tétrahydropyridine de formule (II) 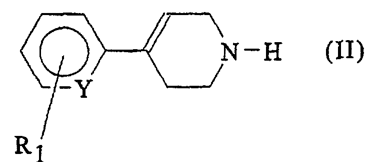 dans laquelle Y et R₁ sont tels que définis pour les composés (I) dans la revendication 1, avec un composé de formule (III) 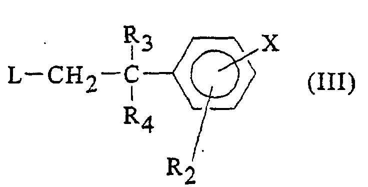 dans laquelle R₂, R₃, R₄ et X sont tels que définis pour les composés (I) dans la revendication 1, et L représente un groupe partant ; et
(b) on isole le composé de formule (I) ainsi obtenu et, éventuellement on le transforme en l'un de ses sels ou solvates ou l'un de ses sels d'ammonium quaternaire.

10. Composition pharmaceutique, caractérisée en ce qu'elle contient un composé selon l'une quelconque des revendications 1 à 8 en tant que principe actif.

11. Composition pharmaceutique contenant, en tant que principes actifs, un composé selon l'une quelconque des revendications 1 à 8 et un composé indiqué dans le traitement symptomatique de la démence sénile du type Alzheimer (DAT) ou leurs sels pharmaceutiquement acceptables.

## Claims

1. Compound of formula (I): 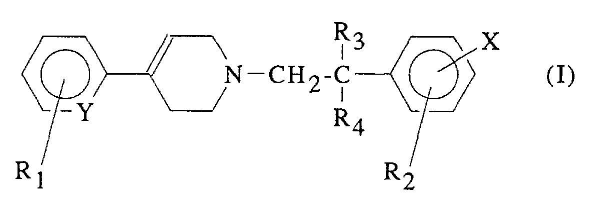 in which:
Y is -CH- or -N-;
R₁ is hydrogen, a halogen or a CF₃, (C₁-C₄)alkyl or (C₁-C₄)alkoxy group;
R₂ is a methyl or ethyl group;
R₃ and R₄ are each hydrogen or a (C₁-C₃)alkyl; and
X is:
(a) a (C₁-C₆)alkyl, a (C₁-C₆)alkoxy, a (C₃-C₇)carboxyalkyl, a (C₁-C₄)alkoxycarbonyl(C₁-C₆)alkyl, a (C₃-C₇)carboxyalkoxy or a (C₁-C₄)alkoxycarbonyl(C₁-C₆)alkoxy;
(b) a radical selected from (C₃-C₇)cycloalkyl, (C₃-C₇)cycloalkoxy, (C₃-C₇)cycloalkylmethyl, (C₃-C₇)cycloalkylamino and cyclohexenyl, it being possible for said radical to be substituted by a halogen, hydroxyl, (C₁-C₄)alkoxy, carboxyl, (C₁-C₄)alkoxycarbonyl, amino or mono- or di-(C₁-C₄)alkylamino; or
(c) a group selected from a phenyl, phenoxy, phenylamino, N-(C₁-C₃)alkylphenylamino, phenylmethyl, phenylethyl, phenylcarbonyl, phenylthio, phenylsulfonyl, phenylsulfinyl and styryl, it being possible for said group to be monosubstituted or polysubstituted on the phenyl group by a halogen, CF₃, (C₁-C₄)alkyl, (C₁-C₄)alkoxy, cyano, amino, mono- or di-(C₁-C₄)alkylamino, (C₁-C₄)acylamino, carboxyl, (C₁-C₄)alkoxycarbonyl, aminocarbonyl, mono- or di-(C₁-C₄)alkylaminocarbonyl, amino(C₁-C₄)alkyl, hydroxy(C₁-C₄)alkyl or halogeno(C₁-C₄)alkyl;
and its salts and solvates and its quaternary ammonium salts.

2. Compound according to claim 1 in which Y is a group -CH- and R₁ is CF₃.

3. Compound according to claim 1 in which Y is a nitrogen atom and R₁ is a chlorine atom.

4. Compound according to one of claims 1 to 3 in which X is a (C₁-C₆)alkyl group.

5. Compound according to claim 1 in which X is a group (c) where the phenyl is substituted by 1 to 3 halogens, 1 to 3 CF₃, 1 to 3 (C₁-C₄)alkyl, 1 to 3 (C₁-C₄)alkoxy, 1 to 3 cyano, 1 to 3 amino, 1 to 3 mono- or di-(C₁-C₄)alkylamino, 1 to 3 (C₁-C₄)acylamino, 1 to 3 carboxyl, 1 to 3 (C₁-C₄)alkoxycarbonyl, 1 to 3 aminocarbonyl, 1 to 3 mono- or di-(C₁-C₄)alkylaminocarbonyl, 1 to 3 amino(C₁-C₄)alkyl, 1 to 3 hydroxy(C₁-C₄)alkyl or 1 to 3 halogeno(C₁-C₄)alkyl.

6. Compound according to claim 1 of formula (I'): 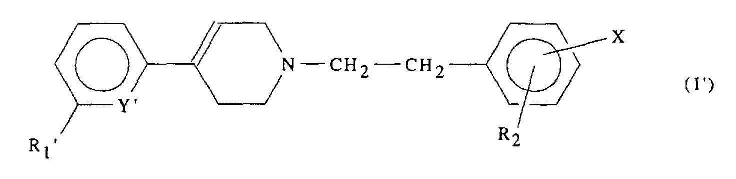 in which R₁' is CF₃ and Y' is CH, or R₁' is Cl and Y' is N, R₂ and X being as defined for the compounds (I) in claim 1, and its salts, solvates and quaternary ammonium salts.

7. Compound according to claim 6 in which X is a (C₁-C₆)alkyl group.

8. Compound according to claim 1 selected from 1-[2-(3,4-diethylphenyl)ethyl]-4-(3-trifluoromethylphenyl)-1,2,3,6-tetrahydropyridine, 1-[2-(3-methyl-4-pentylphenyl)ethyl]-4-(3-trifluoromethylphenyl)-1,2,3,6-tetrahydropyridine, 1-[2-(4-methyl-3-pentylphenyl)ethyl]-4-(3-trifluoromethylphenyl)-1,2,3,6-tetrahydropyridine, 1-[2-(3,4-diethylphenyl)ethyl]-4-(6-chloropyrid-2-yl)-1,2,3,6-tetrahydropyridine and their salts, solvates or quaternary ammonium salts.

9. Method of preparing the compounds of formula (I) according to claim 1, their salts or solvates and their quaternary ammonium salts, characterized in that:
(a) an aryl-1,2,3,6-tetrahydropyridine of formula (II): 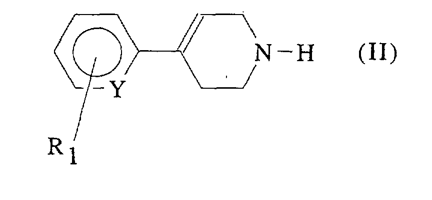 in which Y and R₁ are as defined for the compounds (I) in claim 1, is reacted with a compound of formula (III): 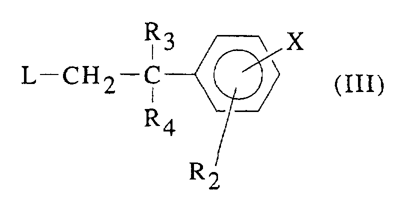 in which R₂, R₃, R₄ and X are as defined for the compounds (I) in claim 1 and L is a leaving group; and
(b) the resulting compound of formula (I) is isolated and optionally converted to one of its salts or solvates or one of its quaternary ammonium salts.

10. Pharmaceutical composition, characterized in that it contains a compound according to any one of claims 1 to 8 as the active principle.

11. Pharmaceutical composition containing, as the active principles, a compound according to any one of claims 1 to 8 and a compound indicated in the symptomatic treatment of senile dementia of the Alzheimer type (DAT), or their pharmaceutically acceptable salts.

## Patentansprüche

1. Verbindung der Formel (I) 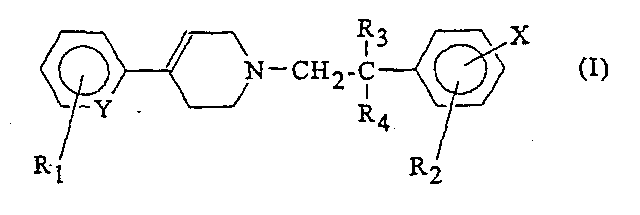 worin
Y -CH- oder -N- darstellt;
R₁ Wasserstoff; Halogen, eine Gruppe CF₃, (C₁-C₄)-Alkyl oder (C₁-C₄)-Alkoxy darstellt;
R₂ eine Methyl- oder Ethylgruppe darstellt;
R₃ und R₄ jeweils Wasserstoff oder (C₁-C₃)-Alkyl darstellen;
X folgendes darstellt:
(a) (C₁-C₆)-Alkyl, (C₁-C₆)-Alkoxy, (C₃-C₇)-Carboxyalkyl, (C₁-C₄)-Alkoxycarbonyl-(C₁-C₆)-alkyl, (C₃-C₇)-Carboxyalkoxy oder (C₁-C₄)-Alkoxycarbonyl-(C₁-C₆)-alkoxy;
(b) einen Rest ausgewählt aus (C₃-C₇)-Cycloalkyl, (C₃-C₇)-Cycloalkyloxy, (C₃-C₇)-cycloalkylmethyl, (C₃-C₇)-Cycloalkylamino und Cyclohexenyl, wobei der Rest durch ein Halogen, Hydroxy, (C₁-C₄)-Alkoxy, Carboxy, (C₁-C₄)-Alkoxycarbonyl, Amino, Mono- oder Di-(C₁-C₄)-Alkylamino substituiert sein kann; oder
(c) eine Gruppe ausgewählt aus Phenyl, Phenoxy, Phenylamino, N-(C₁-C₃)-Alkylphenylamino, Phenylmethyl, Phenylethyl, Phenylcarbonyl, Phenylthio, Phenylsulfonyl, Phenylsulfinyl oder Styryl, wobei die Gruppe an der Phenylgruppe durch ein Halogen, CF₃, (C₁-C₄)-Alkyl, (C₁-C₄)-Alkoxy, Cyano, Amino, Mono- oder Di-(C₁-C₄)-alkylamino, (C₁-C₄)-Acylamino, Carboxy, (C₁-C₄)-Alkoxycarbonyl, Aminocarbonyl, Mono- oder Di-(C₁-C₄)-alkylaminocarbonyl, Amino-(C₁-C₄)-alkyl, Hydroxy-(C₁-C₄)-alkyl oder Halogend-(C₁-C₄)-alkyl mono- oder polysubstituiert sein kann;
sowie ihre Salze und Solvate und ihre quaternären Ammoniumsalze.

2. Verbindung nach Anspruch 1, worin Y eine Gruppe -CH- darstellt und R₁ für CF₃ steht

3. Verbindung nach Anspruch 1, worin Y ein Stickstoffatom und R₁ ein Chloratom ist.

4. Verbindung nach einem der Ansprüche 1 bis 3, worin X eine Gruppe (C₁-C₆)-Alkyl ist.

5. Verbindung nach Anspruch 1, worin X eine Gruppe (c) ist, in der das Phenyl durch 1 bis 3 Halogene, 1 bis 3 CF₃, 1 bis 3 (C₁-C₄)-Alkyl, 1 bis 3 (C₁-C₃)-Alkoxy, 1 bis 3 Cyano, 1 bis 3 Amino, 1 bis 3 Mono- oder Di-(C₁-C₄)-Alkylamino, 1 bis 3 (C₁-C₄)-Acylamino, 1 bis 3 Carboxy, 1 bis 3 (C₁-C₄)Alkoxycarbonyl, 1 bis 3 Aminocarbonyl, 1 bis 3 Mono- oder Di-(C₁-C₄)-alkylaminocarbonyl, 1 bis 3 Amino(C₁-C₄)-alkyl, 1 bis 3 Hydroxy-(C₁-C₄)-alkyl oder 1 bis 3 Halogen-(C₁-C₄)-alkyl substituiert ist

6. Verbindung nach Anspruch 1 der Formel (I') 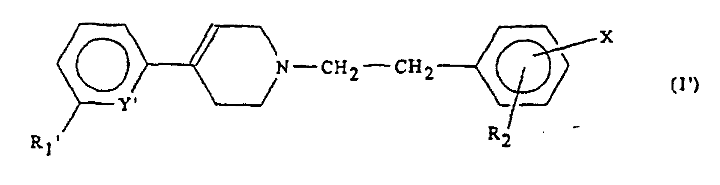 worin R₁' für CF₃ steht und Y' für CH steht oder aber R₁' für Cl steht und Y' für N steht und R₂ und X wie in Anspruch 1 für die Verbindungen (I) definiert sind, sowie ihre Salze und Solvate und quaternären Ammoniumsalze.

7. Verbindung nach Anpruch 6, worin X eine Gruppe (C₁-C₆)-Alkyl ist.

8. Verbindung nach Anspruch 1, ausgewählt aus 1-[2-(3,4-diethylphenyl)ethyl]-4-(3-trifluormethylphenyl)-1,2,3,6-tetrahydropyridin, 1-[2-(3-Methyl-4-pentylphenyl)ethyl]-4-(3-trifluomethylphenyl)-1,2,3,6-tetrahydropyridin, 1-[2-(4-Methyl-3-pentylphenyl)ethyl]-4-(3-trifluormethylphenyl)-1,2,3,6-tetrahydropyridin, 1-[2-(3,4-Diethylphenyl)ethyl]-4-(6-chlorpyrid-2-yl)-1,2,3,6-tetrahydropyridin und ihren Salzen, Solvaten oder quaternären Ammoniumsalzen.

9. Verfahren zur Herstellung der Verbindungen der Formel (I) nach Anspruch 1, ihrer Salze oder Solvate und ihrer quaternären Ammoniumsalze, dadurch gekennzeichnet, dass
(a) ein Aryl-1,2,3,6-tetrahydropyridin der Formel (II) 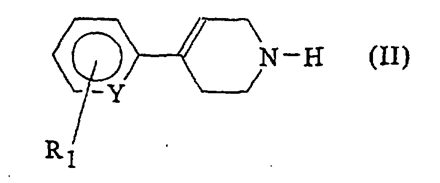 worin Y und R₁ wie in Anspruch 1 für die Verbindungen (I) definiert sind, mit einer Verbindung der Formel (III) 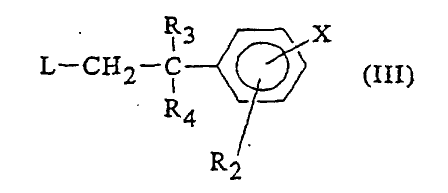 worin R₂, R₃, R₄ und X wie in Anspruch 1 für die Verbindungen (I) definiert sind und L eine austretende Gruppe ist, zur Umsetzung gebracht wird, und
(b) die so erhaltene Verbindung der Formel (I) isoliert und gegebenenfalls in eines ihrer Salze oder Solvate oder eines ihrer quaternären Ammoniumsalze übergeführt wird.

10. Pharmazeutische Zusammensetzung, dadurch gekennzeichnet, dass sie eine Verbindung nach einem der Ansprüche 1 bis 8 als Wirkstoff enthält

11. Pharmazeutische Zusammensetzung, die als Wirkstoffe eine Verbindung nach einem der Ansprüche 1 bis 8 und eine Verbindung, die bei der symptomatischen Behandlung von seniler Demenz vom Alzheimer-Typ (DAT) indiziert ist, oder die pharmazeutisch annehmbaren Salze derselben enthält
